# EUROPEAN PATENT SPECIFICATION

(11) **EP 3 488 828 B1**
(45) Date of publication and mention of the grant of the patent: **02.09.2026**
(21) Application number: 17831095.9
(22) Date of filing: 20.07.2017
(51) Int. Cl.: A61F 5/02, A41D 13/05, A61F 5/01

(54) **SPINE MEMBER FOR ASSISTANCE SUIT, AND ASSISTANCE SUIT**
RÜCKENELEMENT FÜR HILFSGARNITUR UND HILFSGARNITUR
ÉLÉMENT DE COLONNE VERTÉBRALE POUR UNE COMBINAISON D'ASSISTANCE.

(30) Priority: 20.07.2016 JP 2016142209
(43) Date of publication of application: 29.05.2019
(73) Proprietor: UPR Corporation, Yamaguchi 7550032 (JP); Virgo Wave Inc., Tokyo 104-0051 (JP)
(72) Inventor: SAKATA, Kenji, Tokyo 100-0011 (JP); IWAMOTO, Akinori, Tokyo 104-0051 (JP)
(74) Representative: Samson & Partner Patentanwälte mbB
(86) International application number: PCT/JP2017/026265
(87) International publication number: WO 2018/016579

(56) References cited:
- EP-A2- 1 834 613
- GB-A- 740 507
- JP-A- 2001 218 779
- JP-A- 2006 305 290
- JP-A- 2012 040 152
- JP-A- 2013 144 858
- JP-A- 2013 144 858
- JP-A- 2014 090 959
- JP-A- 2015 505 267
- JP-U- H0 648 621
- NL-C2- 1 008 185
- US-A- 5 619 747
- US-A1- 2010 318 010

## Description

### Technical Field

The present invention relates to an assist suit that corrects the posture of the body of the wearer and assists his/her forward bending motion when he/she transports or lifts and lowers baggage, and a spinal member for the assist suit.

### Background Art

There have conventionally been proposed a wearable tool (see, for example, patent literature 1) for correcting the poor posture of the upper body of the wearer and a wearable tool (see, for example, patent literatures 2 to 4) that assists his/her forward bending motion when he/she transports or lifts and lowers baggage.
US 2010/318010 A1 relates to a protective brace for figure skaters.
JP 2013 144858 A relates to a work aid for being worn in a forward-bent working position.
GB 740 507 A relates to orthopraxis appliances.
EP 1 834 613 A2 relates to a spinal orthosis.
US 5 619 747 A relates to an adjustable posterior spinal orthosis.

NL 1 008 185 C2 relates to an orthosis used to correct the position of the spine and discloses the features defined in the preamble of claim 1.

### Related Art Literature

### Patent Literature

Patent Literature 1: Japanese Patent Laid-Open No. 2001-218779
Patent Literature 2: Japanese Patent Laid-Open No. 2002-161414
Patent Literature 3: Japanese Patent Laid-Open No. 2011-188896
Patent Literature 4: Japanese Patent Laid-Open No. 2013-144858

### Disclosure of Invention

### Problem to be Solved by the Invention

For example, the wearable tool disclosed in patent literature 1 is a posture correction wearable tool for the upper body, which includes a back support, shoulder belts, and waist belts and is configured such that the right and left shoulder belts are made to extend through the lower portion of the back support, while being crossed, and then engaged with each other, and the waist belts coupled to the two side edge portions of the lower portion of the back support are engaged with each other. Right and left two plate-like pieces for enhancing the posture correction effect are inserted into the pockets of the back support.

Patent literature 2 discloses an upper body support device including a back pad covering the entire back of the wearer and extending to the back sides of the thigh regions. The upper end of the back pad is fixed to the upper body with shoulder belts. The lower end of the back pad is separated into right and left portions, which are respectively fixed to the right and left thigh regions. This device is configured to exert a force to raise the upper body in forward bending motion by using the repulsion force of an elastic columnar support incorporated in the back pad. In addition, patent literatures 3 and 4 each have proposed an assist tool including an elastic member that extends from the back of the body to the thigh regions to assist the forward bending motion of the wearer.

The wearable tool disclosed in patent literature 1 is worn on only the upper body, and hence provides a comfortable feeling in wearing and does not give much irritation or restriction of daily activities. However, the two plate-like pieces inserted in the back support are only long enough to extend from the back of the wearer to the waist region. Accordingly, this tool cannot be expected to provide the effect of assisting the forward bending motion of the wearer even though it can correct the poor posture such as a hunched-back posture.

In contrast, the upper body support device disclosed in patent literature 2 has the function of assisting forward bending motion but has a problem that the device gives the wearer an uncomfortable feeling of wearing and provides a greater restriction of daily activities, such as body twisting motion, a body tilting motion, and motion of sitting on a chair as the back pad is covering the entire back of the wearer, and the back pad extends to the back sides of the thigh regions.

The assist tools disclosed in patent literatures 3 and 4 each provide a more comfortable feeling to a wearer compared to the prior art technology using the back pad as disclosed in patent literature 2. However, the assist tools disclosed in patent literatures 3 and 4 still cannot reduce restriction of daily activities of the wearer such as motion of sitting on a chair because the elastic member extends from the back of the body to the thigh regions.

The present invention has been made to solve the above problem, and has as its object to provide an assist suit that achieves both a posture correction effect and a forward bending assisting effect, provides a more comfortable feeling of wearing, and provides less restriction of daily activities.

### Means of Solution to the Problem

The present invention provides a spinal member according to claim 1.

### Effects of the Invention

The present invention can provide an assist suit that achieves both a posture correction effect and a forward bending assisting effect, provides a more comfortable feeling of wearing, and provide less restriction of daily activities.

### Brief Description of Drawings

Fig. 1 is a view showing an example of the configuration (rear view) of an assist suit;
Fig. 2 is a view showing another example of the configuration (rear view) of the assist suit;
Fig. 3 is a view showing an example of the spinal member of the assist suit;
Fig. 4 is a view showing an example of the configuration (rear and side views) of the assist suit worn by the wearer;
Fig. 5 is a view showing an operation example (side view) of the assist suit when the wearer makes forward bending motion;
Fig. 6 is a view showing another operation example (side view) of the assist suit when the wearer makes forward bending motion;
Fig. 7 is a view showing an example of a spinal member having laterally separated portions;
Fig. 8 is a view showing another example of the spinal member having laterally separated portions;
Fig. 9 is a view showing an example of a spinal member having a protruding portion;
Fig. 10 is a view showing an example of a spinal member having both laterally separated portions and a protruding portion;
Fig. 11 is a view showing an example of a spinal member having a protruding portion with a groove; and
Fig. 12 is a view showing another example of the spinal member having a protruding portion with a groove.

### Best Mode for Carrying Out the Invention

An embodiment of the present invention will be described next with reference to the accompanying drawings.

### <Assist Suit>

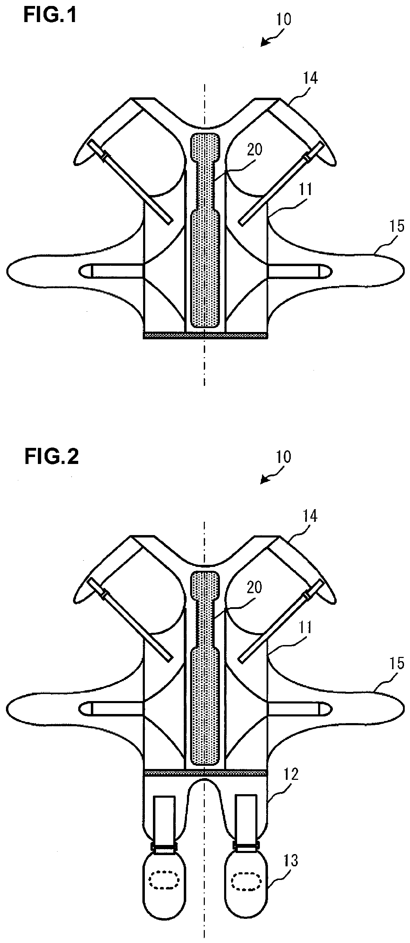

An assist suit including a spinal member for the assist suit according to an embodiment of the present invention will be described first with reference to Figs. 1 and 2.

An assist suit 10 in Fig. 1 includes a back region wearing unit 11 worn on the back region of the wearer. The back region wearing unit 11 includes shoulder belts 14 to be fixed to the back region of the wearer and waist belts 15. The back region wearing unit 11 is preferably formed from a cloth material, a knitted fiber material, or the like that has certain flexibility and extensibility for improvement in comfort of wearing. However, they are not exhaustive. The back region wearing unit 11 may be formed from a combination of a cloth material and an extensible belt-like member.

The shoulder belts 14 and the waist belts 15 each are configured to be adjustable in length with engaging members such as a belt, a buckle, and a surface fastener and adjustable in degree of contact between the back region wearing unit 11 and the wearer.

The back region wearing unit 11 includes an elongated spinal member 20 extending from the upper portion of the back region of the wearer to the thigh region. The spinal member 20 is formed from a member that bends along the back region of the wearer and has elasticity in the bending direction. The spinal member 20 and the back region wearing unit 11 are in tight contact with each other and move together. This makes it possible to correct the posture of the wearer and assist the forward bending motion of the wearer.

The assist suit 10 in Fig. 2 includes a buttock region wearing unit 12 worn on the buttock regions of the wearer and thigh region wearing units 13 worn on the right and left thigh regions of the wearer in addition to the components of the assist suit in Fig. 1. The buttock region wearing unit 12 is coupled to the back region wearing unit 11 and the thigh region wearing units 13. Like the back region wearing unit 11, the buttock region wearing unit 12 and the thigh region wearing units 13 each are preferably formed from a cloth material, a knitted fiber material, or the like that has certain flexibility and extensibility for improvement in feeling of wearing. However, they are not exhaustive. These wearing units each may be formed from a combination of a cloth material and an extensible belt-like member.

The thigh region wearing units 13 are formed from cylindrical members having extensibility to be fixed to the right and left thigh regions of the wearer, and are fixed to the thigh regions by being circumferentially fastened. The thigh region wearing units 13 each may be formed from a belt-like member having an engaging means.

The buttock region wearing unit 12 is formed from a member having higher extensibility than the back region wearing unit 11. The buttock region wearing unit 12 formed from a member having high extensibility is coupled to the thigh region wearing units 13 fixed to the thigh regions of the wearer. Accordingly, when the wearer makes forward bending motion, the buttock region wearing unit 12 expands and contracts to assist the wearer to raise his/her upper body upon making forward bending motion.

The buttock region wearing unit 12 may be detachably coupled to the back region wearing unit 11. Detachable coupling makes it easy to adjust the extensibility of the members forming the buttock region wearing unit 12.

The lower end of the buttock region wearing unit 12 may be separated into right and left portions, which may be respectively coupled to the right and left thigh region wearing units 13.

### <Spinal Member>

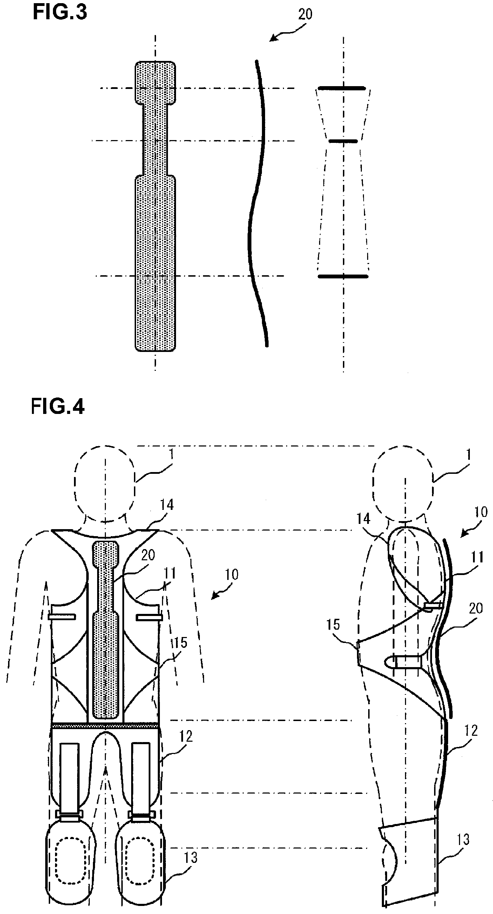

The spinal member 20 of the assist suit 10 will be described with reference to Fig. 3.

The spinal member 20 in this configuration example is formed from a member that has a vertically long shape extending from the upper portion of the back region of the wearer to the buttock region, bends along the back region of the wearer, and has elasticity in the bending direction. A member forming the spinal member 20 is a member formed from a metal material, resin material, or the like having elasticity in the bending direction. However, the member is not limited to these materials.

The spinal member 20 is formed from a plate-like member having different widths in the longitudinal direction. In the configuration example in Fig. 2, a part of the spinal member which is located above a middle portion in the longitudinal direction, for example, a part near the shoulder blade of the back region of the wearer is narrower than the remaining parts. This makes it possible to configure the spinal member 20 so as not to interfere with the twisting motion of the upper body of the wearer as much as possible while maintaining the elastic force of the spinal member.

### <Wearing of Assist Suit>

Fig. 4 shows a state in which the assist suit 10 described with reference to Fig. 2 is being worn. The state in which the assist suit 10 in Fig. 1 is being worn is the same as that shown in Fig. 2 except that the suit does not include the buttock region wearing unit 12 and the thigh region wearing units 13, and hence a description of the state will be omitted.

For the sake of brevity, the side view of Fig. 4 shows a state in which the spinal member 20 is separated from the back region wearing unit 11. However, the spinal member 20 according to the present invention is in tight contact with the back region wearing unit 11, and the back region wearing unit 11 and the spinal member 20 move together. This applies to other drawings.

As shown in the rear view of Fig. 4, the back region wearing unit 11 of the assist suit 10 according to the present invention is fixed to the back region of a wearer 1 with the shoulder belts 14 and the waist belts 15. The buttock region wearing unit 12 is coupled to the back region wearing unit 11 and is coupled to the thigh region wearing units 13 fixed to the thigh regions of the wearer 1. In addition, as shown in the side view of Fig. 4, the spinal member 20 according to the present invention bends along the back region of the wearer 1, and the back region wearing unit 11 is in tight contact with a region extending from the upper portion of the back region of the wearer 1 to the buttock region. The poor posture of the wearer 1, such as a hunched-back posture, can be corrected by fixing the back region wearing unit 11 including the spinal member 20 to the back region of the wearer 1 with the shoulder belts 14 and the waist belts 15.

The buttock region wearing unit 12 is coupled to the back region wearing unit 11 and is coupled to the thigh region wearing units 13 fixed to the right and left thigh regions of the wearer 1. The thigh region wearing units 13 are formed from extensible cylindrical members to be fixed to the right and left thigh regions of the wearer, and each are configured to circumferentially fasten the upper and lower portions of the knee region of the wearer. In this case, the thigh region wearing units 13 may be fixed to the thigh regions of the wearer 1 while the buttock region wearing unit 12 is stretched. Maintaining the stretched state of the buttock region wearing unit 12 in advance while the wearer 1 is in an upright state can increase the extension/contraction force of the buttock region wearing unit 12 when the wearer 1 makes forward bending motion.

According to the configuration in Fig. 4, the thigh region wearing units 13 are formed from the extensible cylindrical members to be fixed to the right and left thigh regions of the wearer. However, the thigh region wearing units 13 each may be formed from a belt-like member including an engaging means. In this case, each thigh region wearing unit 13 is fixed by fastening the upper and lower portions of the knee region of the wearer with the belt-like member.

### <Operation of Assist Suit>

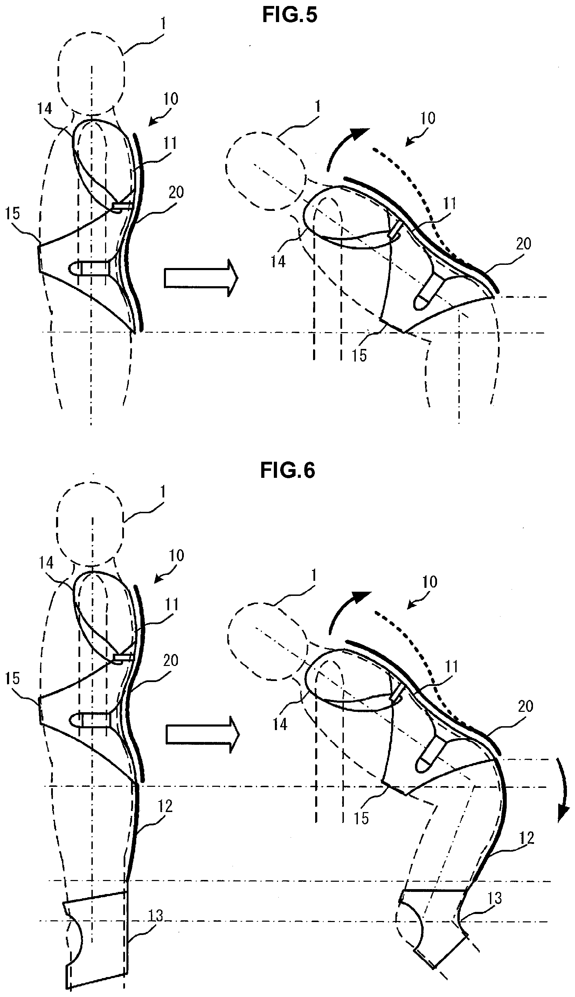

The operation of the assist suit 10 when the wearer 1 of the assist suit 10 makes forward bending motion will be described with reference to Figs. 5 and 6.

Referring to Fig. 5, when the wearer 1 wearing the assist suit 10 makes forward bending motion, the back region wearing unit 11 is fixed to the back region of the wearer 1 with the shoulder belts 14 and the waist belts 15, and hence moves together with the forward bending motion of the wearer 1. At this time, because the upper body of the wearer 1 bends from the waist region of the wearer 1 in the forward direction, the spinal member 20 in tight contact with the back region wearing unit 11 of the wearer 1 is also forcibly bent in the forward bending direction of the wearer 1.

In this case, because the spinal member 20 is in tight contact with the back region wearing unit 11 throughout the region from the upper portion of the back region of the wearer 1 to the buttock region, an elastic force acts on the spinal member 20 in the bending direction, with the buttock region of the wearer 1 serving as a starting point. This elastic force acting on the spinal member 20, starting from the buttock region, will produce a force acting on the shoulder belts 14 of the back region wearing unit 11 in tight contact with the spinal member 20 so as to assist the wearer 1 bent forward to raise his/her upper body. In this manner, the spinal member 20 in tight contact with the region from the upper portion of the back region of the wearer 1 to the buttock region can achieve both the posture correction effect and the forward bending assisting effect. The spinal member 20 does not reach the thigh regions of the wearer 1, and hence provides less restriction of daily activities of the wearer, such as motion of sitting on a chair.

Fig. 6 shows an example of the operation of the assist suit 10 when the wearer 1 wearing the assist suit 10 in Fig. 2 makes forward bending motion. The assist suit 10 in Fig. 2 includes the buttock region wearing unit 12 worn on the buttock regions of the wearer and the thigh region wearing units 13 worn on the right and left thigh regions of the wearer in addition to the components of the assist suit in Fig. 1. Including the buttock region wearing unit 12 and the thigh region wearing units 13 can further enhance the forward bending motion assisting effect as compared with the cases shown in Figs. 1 and 5.

The buttock region wearing unit 12 is coupled to the thigh region wearing units 13 fixed to the thigh regions of the wearer 1. Accordingly, when the wearer 1 makes forward bending motion, the buttock region wearing unit 12 is stretched from the thigh region wearing units 13 as starting points. When the buttock region wearing unit 12 is stretched, a force that causes the buttock region wearing unit 12 to contract from the thigh region wearing units 13 as starting points acts on the back region wearing unit 11 to which the buttock region wearing unit 12 is coupled. As a consequence, the extension/contraction force of the buttock region wearing unit 12, with the thigh region wearing units 13 serving as starting points, generates a force acting on the shoulder belts 14 of the back region wearing unit 11 to assist the wearer 1 bending forward to raise his/her upper body. As also described with reference to Fig. 4, fixing the thigh region wearing units 13 to the thigh regions of the wearer 1 while the buttock region wearing unit 12 is stretched can further enhance the assisting force. The buttock region wearing unit 12 is formed from a highly extensible member, and hence provides less restriction of daily activities of the wearer 1, such as motion of sitting on a chair.

According to the configuration example in Fig. 6, the thigh region wearing units 13 are formed from the extensible cylindrical members to be fixed to the right and left thigh regions of the wearer and configured to circumferentially fasten the upper and lower portions of the knee regions of the wearer. This configuration makes it difficult for the thigh region wearing units 13 to shift upward when the wearer makes forward bending motion, thereby enhancing the forward bending motion assisting effect.

The extension/contraction force of the buttock region wearing unit 12 coupled to the back region wearing unit 11 and the thigh region wearing units 13 can achieve a higher forward bending motion assisting effect than in the case of using only the spinal member 20 in Fig. 5. The highly extensible thigh region wearing units 13 provide much less restriction of daily activities of the wearer 1 than that of the conventional elastic member.

As described above, the assist suit according to this embodiment of the present invention can achieve both the effect of correcting the posture of the wearer and the forward bending assisting effect with the spinal member that is in tight contact with the region from the upper portion of the back region of the wearer to the thigh regions. In addition, further including the buttock region wearing unit and the thigh region wearing units can further enhance the forward bending motion assisting effect with the extension/contraction force of the buttock region wearing unit coupled to the thigh region wearing units.

### <Configuration of Another Spinal Member>

Fig. 3 exemplarily shows the configuration in which the spinal member 20 is formed from the plate-like member having different widths in the longitudinal direction, and a part of the spinal member which is located above the middle portion in the longitudinal direction, for example, a part near the shoulder blade of the back region of the wearer 1 is narrower than the remaining parts. However, a portion of the spinal member 20 in the longitudinal direction may be laterally separated along an axis corresponding to the spine of the wearer 1.

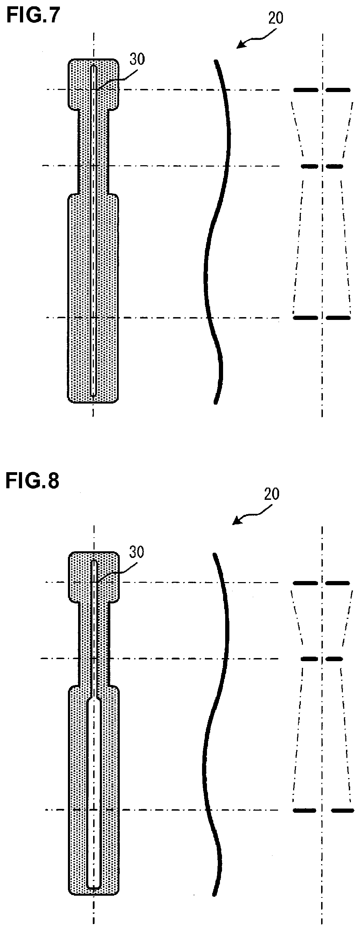

Fig. 7 shows an example of the spinal member 20 having a portion 30 in which the spinal member 20 is laterally separated in the longitudinal direction. Having a laterally separated spinal member 20 makes it easy for the wearer to twist the spinal member 20. This makes it possible to provide the spinal member 20 that less interferes with the twisting motion of the upper body of the wearer 1 as compared with the configuration in Fig. 2. In addition, because the spinal member 20 is being laterally separated along the axis corresponding to the spine, there is no need to concern about a force degradation in the elastic force of the spinal member 20.

As shown in Fig. 8, the portion 30 laterally separating the spinal member may be configured to extend from the upper portion of the back region of the wearer 1 to the buttock region such that a portion corresponding to the buttock region of the wearer 1 is wider than the remaining portion. This configuration can prevent the spinal member 20 from coming into contact with the spine of the wearer 1 at the buttock region of the wearer 1, thereby providing an assist suit that provide a more comfortable feeling of wearing.

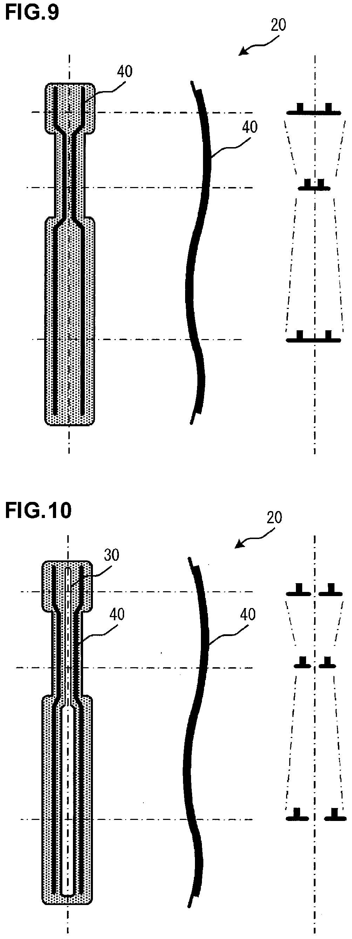

As shown in Fig. 9, the spinal member 20 includes protruding portions 40 extending in the longitudinal direction on the two sides of an axis corresponding to the spine of the wearer 1. Including the protruding portions 40 can increase the elastic force of the spinal member 20 while reducing the width of the spinal member 20. Although at least one protruding portion 40 is required on each of the right and left sides, a plurality of protruding portions 40 may be provided on each of the right and left sides.

As shown in Fig. 10, the spinal member 20 may include both the portion 30 laterally separating the spinal member 20 and the protruding portions 40 extending in the longitudinal direction. This configuration can provide an assist suit that provides a more comfortable feeling of wearing and has a greater assisting effect for forward bending motion.

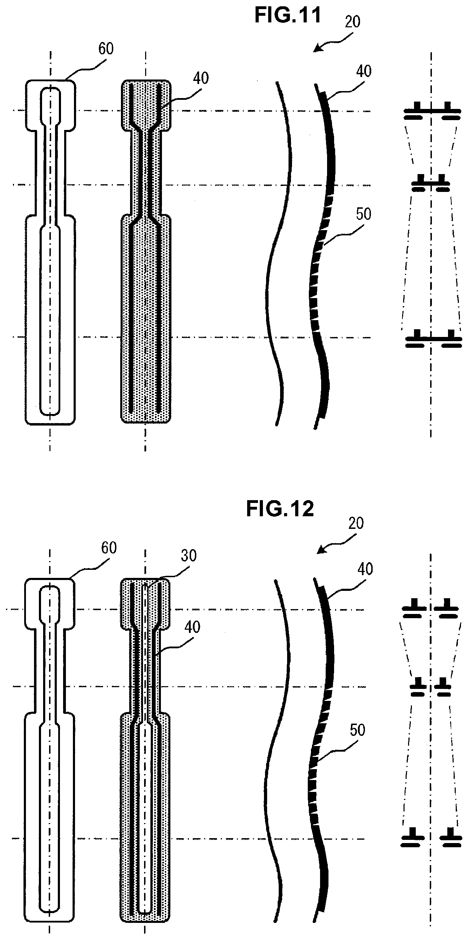

The protruding portions 40 of the spinal member 20 may include a plurality of grooves which cross the protruding portions 40 and open outward from the spine region side of the wearer 1. The spinal member 20 may further include a second spinal member 60 that is formed from a member having elasticity in the bending direction, and the second spinal member 60 comes into tight contact with the spinal member 20 from the spine region side of the wearer 1. Figs. 11 and 12 each show an example of the configuration of the spinal member 20 including the second spinal member 60 in addition to a corresponding one of the configurations in Figs. 9 and 10, with the protruding portions 40 having a plurality of grooves 50 open outward.

According to each of the configuration examples of the spinal members 20 described with reference to each of Figs. 3, 7, and 10, the spinal member 20 is formed from one plate-like member. With such a configuration, when the elastic force of the member forming the spinal member 20 is too strong as compared with the muscle force of the wearer 1, the wearer may experience difficulty in making forward bending motion itself, resulting in uncomfortable feeling of wearing. In contrast to this, when the elastic force of the member forming the spinal member 20 is too weak, a forward bending motion assisting effect may not be obtained.

The spinal members 20 in Figs. 11 and 12 each present an example of the configuration of a spinal member for solving the above situation. That is, the protruding portions 40 of the spinal member 20 have the plurality of grooves 50 that cross the protruding portions 40 and are open outward from the back region side of the wearer 1. This makes it easy for the wearer 1 to make forward bending motion.

Letting the protruding portions 40 have the grooves 50 will weaken the elastic force of the spinal member 20. In order to compensate for this, therefore, the spinal member 20 further include the second spinal member 60 formed from a member having elasticity in the bending direction, which is in tight contact with the spinal member 20 from the back region side of the wearer 1. Using a plurality of such spinal members allows easy adjustment of the elastic forces of the spinal members depending on the muscle force of the wearer 1. In addition, the second spinal member 60 may be formed by punching through a portion of a plate-like spinal member to adjust the elastic force, as exemplarily shown in Figs. 11 and 12.

### Reference Signs List

1...wearer, 10...assist suit, 11...back region wearing unit, 12...buttock region wearing unit, 13...thigh region wearing unit, 14...shoulder belt, 15...waist belt, 20...spinal member

## Claims

1. A spinal member (20) for an assist suit worn by a wearer, the spinal member comprising a first plate-like member having a vertically long shape and configured to extend from a back region of the wearer to a buttock region, bend along the back region of the wearer, and have elasticity in a bending direction,
the spinal member further comprises at least two protruding portions (40) formed on a surface of the first plate-like member to increase the elastic force of the spinal member, the protruding portions extending on two sides of an axis in the longitudinal direction of the first plate-like member,
the first plate-like member includes a part of a portion between one of the end portions and a middle portion in a longitudinal direction of the first plate-like member, the part being narrower than remaining parts of the first plate-like member, wherein the part which is narrower than the remaining parts of the portion of the first plate-like member is configured to be located between shoulder blades of the back region of the wearer when the assist suit is worn by the wearer,
**characterized in that**
the distance between the at least two protruding portions in the part being narrower than the remaining parts of the first plate-like member is narrower than the distances between the at least two protruding portions in the remaining parts of the first plate-like member, wherein the remaining parts of the first plate-like member includes at least the one of the end portions and the middle portion in the longitudinal direction of the first plate-like member.

2. The spinal member according to claim 1, wherein the first plate-like member includes a part (30) which is laterally separated into two portions along the axis in the longitudinal direction, and
the at least two protruding portions (40) are formed in the two portions (30) of the first plate-like member which are laterally separated from each other.

3. The spinal member according to claim 2, wherein an interval between the portions (30) which are laterally separated from each other in a region configured to be worn in the buttock region is greater than an interval between the laterally separated portions at other regions.

4. The spinal member according to any one of claims 1-3, **characterized in that**
the spinal member further comprises a second plate-like member (60) coming into contact with the surface opposite the surface with the at least two protruding portions of the first plate-like member, the second plate-like member having a vertically long shape and configured to extend from a back region of the wearer to a buttock region, bend along the back region of the wearer, and have elasticity in a bending direction, wherein the second plate-like member includes a part between one of the end portions and a middle portion in a longitudinal direction of the first and second plate-like members, the part of the portion, which is configured to be located between shoulder blades of the back region of the wearer when the assist suit is worn by the wearer, being narrower than remaining parts of the second plate-like member, and
the at least two protruding portions (40) have a plurality of grooves (50) open to a direction away from the surface of the first plate-like member onto which the at least two protruding portions are attached.

5. An assist suit comprising a back region wearing unit (11) configured to be worn on a back region of a wearer, a buttock region wearing unit (12) coupled to the back region wearing unit and configured to be worn on a buttock region of the wearer, thigh region wearing units (13) configured to be worn on right and left thigh regions of the wearer, and a spinal member (20) which is in tight contact with the back region wearing unit, wherein the back region wearing unit includes shoulder belts and waist belts to be fixed to a back region of the wearer, wherein
the thigh region wearing units (13) are formed from cylindrical or belt-like members coupled to the buttock region wearing unit and having an extensibility so as to be fixed to the right and left thigh regions of the wearer, and
the spinal member (20) is defined in any one of claims 1 to 4.

## Patentansprüche

1. Ein Wirbelsäulenelement (20) für einen von einem Träger getragenen Unterstützungsanzug, wobei das Wirbelsäulenelement ein erstes plattenartiges Element umfasst, das eine vertikal lange Form aufweist und so ausgebildet ist, dass es sich von einem Rückenbereich des Trägers bis zu einem Gesäßbereich erstreckt, sich entlang des Rückenbereichs des Trägers biegt und in einer Biegerichtung elastisch ist,
das Wirbelsäulenelement ferner mindestens zwei vorstehende Abschnitte (40) umfasst, die an einer Oberfläche des ersten plattenartigen Elements ausgebildet sind, um die elastische Kraft des Wirbelsäulenelements zu erhöhen, wobei sich die Vorsprünge zu beiden Seiten einer Achse in Längsrichtung des ersten plattenartigen Elements erstrecken,
das erste plattenartige Element einen Abschnitt eines Teils zwischen einem der Endabschnitte und einem Mittelabschnitt in Längsrichtung des ersten plattenartigen Elements umfasst, wobei dieser Abschnitt schmaler ist als die übrigen Teile des ersten plattenartigen Elements, wobei der Abschnitt, der schmaler ist als die übrigen Teile des Teils des ersten plattenartigen Elements, so ausgebildet ist, dass er sich zwischen den Schulterblättern des Rückenbereichs des Trägers befindet, wenn der Unterstützungsanzug vom Träger getragen wird,
**dadurch gekennzeichnet, dass**
der Abstand zwischen den mindestens zwei vorstehenden Abschnitten in dem Teil, der schmaler ist als die übrigen Teile des ersten plattenartigen Elements, schmaler ist als die Abstände zwischen den mindestens zwei vorstehenden Abschnitten in den übrigen Teilen des ersten plattenförmigen Elements, wobei die übrigen Teile des ersten plattenförmigen Elements mindestens einen der Endabschnitte und den Mittelabschnitt in Längsrichtung des ersten plattenförmigen Elements umfassen.

2. Wirbelsäulenelement nach Anspruch 1, wobei das erste plattenartige Element einen Abschnitt (30) umfasst, der in zwei seitlich sich in Längsrichtung entlang der Achse erstreckende Abschnitte unterteilt ist, und
die mindestens zwei vorstehenden Abschnitte (40) in den beiden seitlich voneinander getrennten Abschnitten (30) des ersten plattenartigen Elements ausgebildet sind.

3. Wirbelsäulenelement nach Anspruch 2, wobei der seitliche Abstand zwischen den voneinander getrennten Abschnitten (30), die in einem Bereich, der zum Tragen im Gesäßbereich ausgelegt ist, größer ist als ein Abstand zwischen den seitlich getrennten Abschnitten in anderen Bereichen.

4. Wirbelsäulenelement nach einem der Ansprüche 1 bis 3, **dadurch gekennzeichnet, dass**
das Wirbelsäulenelement ferner ein zweites plattenartiges Element (60) aufweist, das
mit derjenigen Oberfläche in Kontakt kommt, die der Oberfläche mit den mindestens zwei vorstehenden Abschnitten des ersten plattenartigen Elements gegenüberliegt, wobei
das zweite plattenartige Element eine vertikal lange Form aufweist und so ausgebildet ist, dass es sich von einem Rückenbereich des Trägers bis zu einem Gesäßbereich erstreckt, sich entlang des Rückenbereichs des Trägers biegt und in einer Biegerichtung elastisch ist,
wobei das zweite plattenartige Element einen Abschnitt in Längsrichtung zwischen einem der Endabschnitte und einem Mittelabschnitt des ersten und des zweiten plattenartigen Elements umfasst, wobei der Teil des Abschnitts, der so ausgebildet ist, dass er sich zwischen den Schulterblättern des Rückenbereichs des Trägers befindet, wenn der Unterstützungsanzug vom Träger getragen wird, schmaler ist als die übrigen Teile des zweiten plattenartigen Elements, und
die mindestens zwei vorstehenden Abschnitte (40) eine Vielzahl von Nuten (50) aufweisen, die in eine Richtung weg von der Oberfläche des ersten plattenartigen Elements offen sind, an dem die mindestens zwei vorstehenden Abschnitte befestigt sind.

5. Ein Unterstützungsanzug, umfassend eine Rückenbereich-Trageeinheit (11), die so ausgebildet ist, dass sie an einem Rückenbereich eines Trägers getragen wird, eine Gesäßbereich-Trageeinheit (12), die mit der Rückenbereich-Trageeinheit verbunden und so ausgebildet ist, dass sie an einem Gesäßbereich des Trägers getragen wird, Trageeinheiten (13) für den Oberschenkelbereich, die dazu ausgelegt sind, am rechten und linken Oberschenkelbereich des Trägers getragen zu werden, sowie ein Wirbelsäulenelement (20), das in engem Kontakt mit der Trageeinheit für den Rückenbereich steht, wobei die Trageeinheit für den Rückenbereich Schultergurte und Hüftgurte umfasst, die am Rückenbereich des Trägers befestigt werden sollen, wobei
die Trageeinheiten für den Oberschenkelbereich (13) aus zylindrischen oder gurtartigen Elementen gebildet sind, die mit der Trageeinheit für den Gesäßbereich verbunden sind und eine Dehnbarkeit aufweisen, um an den rechten und linken Oberschenkelbereichen des Trägers befestigt zu werden, und
das Wirbelsäulenelement (20) in einem der Ansprüche 1 bis 4 definiert ist.

## Revendications

1. Élément rachidien (20) pour une combinaison d'assistance portée par un utilisateur, l'élément rachidien comprenant un premier élément de type plaque présentant une forme verticalement longue et configuré pour s'étendre d'une région dorsale de l'utilisateur jusqu'à une région fessière, se courber le long de la région dorsale de l'utilisateur, et présenter une élasticité dans une direction de flexion,
l'élément rachidien comprend en outre au moins deux portions saillantes (40) formées sur une surface du premier élément de type plaque pour augmenter la force élastique de l'élément rachidien, les portions saillantes s'étendant sur deux côtés d'un axe dans la direction longitudinale du premier élément de type plaque,
le premier élément de type plaque comprend une partie d'une portion entre l'une des portions d'extrémité et une portion centrale dans une direction longitudinale du premier élément de type plaque, la partie étant plus étroite que les parties restantes du premier élément de type plaque, dans lequel la partie qui est plus étroite que les parties restantes de la portion du premier élément de type plaque est configurée pour être située entre les omoplates de la région dorsale de l'utilisateur lorsque la combinaison d'assistance est portée par l'utilisateur, **caractérisé en ce que**
la distance entre les au moins deux portions saillantes dans la partie plus étroite que les parties restantes du premier élément de type plaque est inférieure aux distances entre les au moins deux portions saillantes dans les parties restantes du premier élément de type plaque, dans lequel les parties restantes du premier élément de type plaque comprennent au moins celle parmi les portions d'extrémité et la partie centrale dans la direction longitudinale du premier élément de type plaque.

2. Élément rachidien selon la revendication 1, dans lequel le premier élément de type plaque comprend une partie (30) qui est séparée latéralement en deux portions le long de l'axe dans la direction longitudinale, et
les au moins deux portions saillantes (40) sont formées dans les deux portions (30) du premier élément de type plaque qui sont séparées latéralement l'une de l'autre.

3. Élément rachidien selon la revendication 2, dans lequel un intervalle entre les portions (30) qui sont séparées latéralement l'une de l'autre dans une région configurée pour être portée dans la région fessière est supérieur à un intervalle entre les portions séparées latéralement dans d'autres régions.

4. Élément rachidien selon l'une quelconque des revendications 1 à 3, **caractérisé en ce que**
l'élément rachidien comprend en outre un deuxième élément de type plaque (60) en contact avec la surface opposée à la surface présentant les au moins deux portions saillantes du premier élément de type plaque, le deuxième élément de type plaque présentant une forme verticalement longue et configuré pour s'étendre d'une région dorsale de l'utilisateur jusqu'à une région fessière, se courber le long de la région dorsale de l'utilisateur, et présenter une élasticité dans une direction de flexion,
dans lequel le deuxième élément de type plaque comprend une partie entre l'une des portions d'extrémité et une partie centrale dans une direction longitudinale des premier et deuxième éléments de type plaque, la partie de la portion, qui est configurée pour être située entre les omoplates de la région dorsale de l'utilisateur lorsque la combinaison d'assistance est portée par l'utilisateur, étant plus étroite que des parties restantes du deuxième élément de type plaque, et
les au moins deux portions saillantes (40) présentent une pluralité de rainures (50) ouvertes dans une direction éloignée de la surface du premier élément de type plaque sur lequel les au moins deux portions saillantes sont fixées.

5. Combinaison d'assistance comprenant une unité de port de région dorsale (11) configurée pour être portée sur une région dorsale d'un utilisateur, une unité de port de région fessière (12) couplée à l'unité de port de région dorsale et configurée pour être portée sur une région fessière de l'utilisateur, des unités de port de région de cuisse (13) configurées pour être portées sur des régions de cuisse droite et gauche de l'utilisateur, et un élément rachidien (20) qui est en contact étroit avec l'unité de port de région dorsale, dans laquelle l'unité de port de région dorsale comprend des sangles d'épaule et des sangles de taille à fixer à une région dorsale de l'utilisateur, dans laquelle
les unités de port de région de cuisse (13) sont formées à partir d'éléments cylindriques ou de type sangle couplés à l'unité de port de région fessière et présentant une extensibilité de sorte à être fixées aux régions de cuisse droite et gauche de l'utilisateur, et
l'élément rachidien (20) est défini selon l'une quelconque des revendications 1 à 4.
